Europäisches Patentamt

European Patent Office

Office européen des brevets

(19)

(11) Veröffentlichungsnummer: **0 039 852
B1**

(12) EUROPÄISCHE PATENTSCHRIFT

(45) Veröffentlichungstag der Patentschrift:
04.09.85

(21) Anmeldenummer: 81103310.9

(22) Anmeldetag: 02.05.81

(51) Int. Cl.⁴: **C 07 C 17/38,** C 07 C 19/08,
F 01 K 25/08, C 23 C 16/34

(54) Verfahren und Anlage zur Ausführung eines Rankine-Prozesses.

(30) Priorität: 08.05.80 DE 3017531

(43) Veröffentlichungstag der Anmeldung:
18.11.81 Patentblatt 81/46

(45) Bekanntmachung des Hinweises auf die Patenterteilung:
04.09.85 Patentblatt 85/36

(84) Benannte Vertragsstaaten:
AT BE CH DE FR GB IT LI LU NL SE

(56) Entgegenhaltungen:
DD - A - 93 685
DE - A - 2 310 749
DE - A - 2 511 842
DE - A - 2 615 663
DE - A - 2 833 586
DE - B - 1 493 354

(73) Patentinhaber: Kali-Chemie Aktiengesellschaft,
Postfach 220 Hans-Böckler-Allee 20,
D-3000 Hannover 1 (DE)

(72) Erfinder: Rudolph, Werner Dipl.-Chem., Dr.rer.nat.,
Oderstrasse 38, D-3000 Hannover 73 (DE)
Erfinder: Fernschild, Günter, Am Asphaltberge 8,
D-3000 Hannover 81 (DE)

## Beschreibung

Die Bemühungen zur Erschliessung neuer Energiequellen befassen sich unter anderem mit der Rückgewinnung und Nutzung vorhandener Abfallenergien durch Anwendung moderner Technologien. Oft handelt es sich jedoch um Wärme niederer Temperatur im Bereich von z.B. 20°C bis 300°C, die in grossen Mengen noch ungenutzt an die Umgebung abgegeben wird. Zu diesen Abwärmeströmen gehören die Abgase industrieller Umwandlungsprozesse und Kühlwasserströme. Eine Umwandlung dieser niedergradigen Abwärme in elektrische Energie war in der Vergangenheit mit den bekannten Technologien wirtschaftlich nicht möglich.

In konventionellen Wärmekraftanlagen erfolgt die Energieumwandlung Wärme/Strom mit dem Arbeitmedium Wasser in flüssiger Form und als Dampf. Der Einsatz von Wasser für diesen Zweck ist aber nur vorteilhaft, wenn die angebotene Wärme bei Temperaturen über etwa 250°C anfällt. Bei Temperaturen zwischen z.B. 80 und 250°C ist Wasser (Wasserdampf) als Arbeitsmittel nicht mehr geeignet, da es z.B. wegen seines hohen spezifischen Volumens zu unverhältnismässig grossen und teuren Apparaten und Maschinen führt.

Theoretische Überlegungen und Berechnungen führten allgemein zu der Erkenntnis, dass insbesondere organische Flüssigkeiten mit Siedetemperaturen wesentlich unterhalb derjenigen des Wassers und mit geringen spezifischen Volumina für dieses Einsatzgebiet besonders geeignet sind, wenn sie neben den oben genannten Forderungen noch folgende Kriterien erfüllen:

nicht brennbar, untoxisch, thermisch stabil, chemisch inert und preiswert.

Als Arbeits- und Kreislaufmittel, die den meisten der oben genannten Forderungen entsprechen, eignen sich zahlreiche Fluor-Chlor-Kohlenwasserstoffe. Dieser Einsatz ist z.B. aus DE-A- 25 11 842 bekannt. Aus dem Angebot an Fluor-Chlor-Kohlenwasserstoffen muss von dem Konstrukteur dasjenige ausgewählt werden, das für den vorgesehenen Temperaturbereich die besten Voraussetzungen bietet, wie z.B. $CFCl_3$ (R11), $CF_2Cl_2$ (R12), $CHF_2Cl$ (R22), $CF_2Cl–CFCl_2$ (R113), $CF_2Cl–CF_2Cl$ (R114), $CF_3–CF_2Cl$ (R115) u.a. Ihre technische Verwendbarkeit haben diese FKW's seit langem als Kältemittel in Kühlaggregaten im grossen Umfang bewiesen.

Der Einsatz von Fluor-Chlor-Kohlenwasserstoffen als Arbeitsmedium in Rankine-Prozessen (Expansionsprozessen) hinsichtlich der Energie-Ausbeute ist wie bei anderen Arbeitsmitteln – z.B. Wasser – aber um so besser, je grösser die Temperaturdifferenz zwischen Verdampfungs- und Kondensationstemperatur ist. Die vorzugsweise einzusetzenden FKW R 11, R 12, R 22, R 113, R 114 und R 155 neigen jedoch in dem Temperaturbereich von 80–250°C in Anwesenheit von Stahl und/ oder Stahllegierungen, wie sie im Kraftwerkbau verwendet werden, sich mit steigender Temperatur mehr oder weniger stark zu zersetzen.

So werden diesbezüglich im Matheson-Gas-Data-Book folgende Angaben gemacht: Zersetzungsrate R 11 bei 200°C

in Stahl: 2%/Jahr
R 113: 6%/Jahr

Im «Technical Bulletin» B2 (1957) – Freon® (Du Pont) werden in der Tabelle 1 – Thermische Stabilität von Freon®-Verbindungen – nachstehende Stabilitätsgrenztemperaturen angegeben:

| Verbindung | Maximale Temperatur bei einer ständigen Einwirkung in Gegenwart von Oil/Stahl und Kupfer °C |
|---|---|
| R 11 | 107 |
| R 113 | 107 |
| R 12 | 121 |
| R 114 | 121 |
| R 22 | 135–149 |
| R 13 | 149 |

Die Angaben beziehen sich im allgemeinen auf «statische» Versuche, d.h. der Fluor-chlor-Kohlenwasserstoff sowie die Metallprobe wurden in einem geschlossenen Behälter verschiedenen Prüf-Temperaturen ausgesetzt.

Von entscheidendem Einfluss auf die thermische Beständigkeit von FKW ist der eingesetzte Werkstoff: Im Matheson-Gas-Data-Book wird für die Verbindungen R 11 – 12 – 22 – 113 – 114 bei hohen Temperaturen nachstehende Reihenfolge der Metalle mit abnehmender Zersetzung angegeben: (max. Zersetzung) Silber > Messing > Bronze > Aluminium > Stahl 1340 > Kupfer > Nickel > Stahl 18–8 > Inconel® (min. Zers.).

Für einen technischen Einsatz von Fluor-chlor-Kohlenwasserstoffen in Rankine-Prozessen bedeutet das, dass ein dauerhafter und unproblematischer Betrieb in Wärme-Kraftanlagen nur zu erreichen ist, wenn

– entweder die Arbeitstemperaturen in solchen Systemen – bei Verwendung von C-Stahl (z.B. St 39) – sehr niedrig gehalten werden, was die Wirtschaftlichkeit einer solchen Anlage stark einschränkt

– oder teure Stahllegierungen und/oder Sonderwerkstoffe, wie z.B. Nickel, eingesetzt werden. Auch in diesem Fall wird die Wirtschaftlichkeit infolge der hohen Investitionskosten stark beeinträchtigt.

Von entscheidender Bedeutung ist, dass solche «Abwärme-Rückgewinnungsanlagen» lange Zeiträume störungsfrei und möglichst wartungsfrei arbeiten. Das kann aber nur dann erreicht werden, wenn eine Thermolyse des Arbeitsmediums «Fluor-chlor-Kohlenwasserstoff» vermieden wird bzw. Thermolyseprodukte aus dem Kreislauf entfernt werden. Unter der Bezeichnung «Thermolyse» ist zu verstehen, dass bei hohen Temperaturen das Arbeitsmittel thermisch zersetzt wird, wobei einmal die aciden Zersetzungsprodukte Chlorid und Fluorid (in der Form von Chlorwasserstoff und Fluorwasserstoff evtl. auch Chlor und Fluor) gebildet werden und zum anderen zahlreiche organische halogenierte Nebenprodukte entstehen.

Als besonders kritisch für das Verfahren in thermisch hochbelasteten FKW-Kreislaufen ist die durch einen Rest-Wasser-Gehalt im Arbeitsmedium bedingte Hydrolyse der FKW's, die zu HCl und HF führt. Diese Halogenwasserstoffsäuren verursachen erhebliche Korrosionen. Darüber hinaus können diese Eisen-Halogen-Verbindungen, wie z.B. $FeF_3$, $FeCl_3$ als Katalysatoren für Isomerisierungs- und Disproportionierungsreaktion mit FKW wirken, was zu einem veränderten thermodynamischen und chemischen Verhalten des jeweilig eingesetzten Fluor-Chlor-Kohlenwasserstoffes führen kann.

Überraschenderweise und unerwartet ist ein Verfahren gefunden worden, das die thermische Stabilität von Fluor-Chlor-Kohlenwasserstoffen in Rankine-Kreisprozessen erheblich verbessert, und/oder Korrosionsschäden in thermisch hochbelasteten, aliphatischen Fluor-Chlor-Kohlenwasserstoff als Kreislaufmittel enthaltenden Apparaturen vermeidet, das dadurch gekennzeichnet wird, dass man das Kreislaufmittel in kondensierter Form über ein hochaktives Adsorptionsmittel für Wasser auf Basis von $Al_2O_3$ und/oder $SiO_2$ und ein basisches Adsorptionsmittel für acide Verbindungen leitet.

Die verschiedenen Massnahmen werden wie folgt erläutert:

1. Vollständige Entfernung von Wasser aus dem Arbeitsmedium. Bereits die äusserst geringen Mengen von 2 bis 10 ppm $H_2O$ im FKW führen unter den thermischen Bedingungen des Kreisprozesses, insbesondere im thermisch hochbelasteten Teil einer Wärme-Kraft-Anlage zur Hydrolyse der FKW unter Bildung der aciden Verbindungen HCl und HF. Es ist deshalb von entscheidender Notwendigkeit dem System auch die geringsten Spuren Feuchtigkeit zu entziehen, wobei vorzugsweise $H_2O$-Gehalte im Kreislaufmedium von < 1 ppm einzuhalten sind. Es ist bekannt, Fluor-Kohlenwasserstoffe mit Trockenperlen und Molekularsieben zu behandeln, um den $H_2O$-Gehalt auf die Werte von 2 bis 10 ppm zu senken.

So ist aus der DE-B 1 493 354 die Verwendung eines speziell vorbehandelten Molekularsiebs zur Entfernung von Wasser aus als Kältemittel dienenden halogenierten Methan- und Äthanderivaten bekannt. Kleinere Werte lassen sich im allgemeinen ohne einen erheblichen technischen Aufwand nicht realisieren. Es wurde nun überraschenderweise gefunden, dass hochaktives Adsorptionsmittel auf Basis $Al_2O_3$ und/oder $SiO_2$ die Entfernung von $H_2O$ in einer technischen Anlage unter den Wert von 1 ppm ermöglicht. Als hochaktive Adsorptionsmittel auf Basis $Al_2O_3$ eignen sich insbesondere hochaktives $Al_2O_3$, wobei $Al_2O_3$ für Säulenchromatographie (z.B. von Fa. Woelm) bevorzugt wird. Damit betrifft die Erfindung ein Verfahren zur nahezu absoluten Trocknung des Arbeitsmediums, dadurch gekennzeichnet, dass in den Kreislauf des kondensierten Arbeitsmediums ein hochaktives Adsorptionsmittel eingeschaltet wird, das direkt oder im Bypass durchströmt werden kann. Es steht im Betriebe einer solchen Wärmekraftmaschine offen, die $H_2O$-Adsorption kontinuierlich oder in Intervallen zu betreiben. Auch ist die Grösse und Anzahl der Adsorber frei wählbar.

Im allgemeinen reicht 1 g hochaktives Adsorptionsmittel pro 2 Liter FKW aus. Als besonders vorteilhaft hat sich dieses Trocknungsverfahren bei Befüllen und Anfahren einer Anlage erwiesen. Die bekannten Methoden der Trocknung einer Anlage durch eine Evakuierung ist bei komplizierten technischen Systemen zeitaufwendig und nicht absolut sicher. So können sich z.B. in Leitungsteilen hinter Ventilen, die während der Evakuierung geschlossen gehalten werden müssen, für eine Hydrolyse ausreichende Mengen an Wasser befinden, die im Augenblick des Öffnens in das Arbeitsmedium gelangen. Dieser gefährliche Nachteil wird vermieden, wenn man das Arbeitsmittel in einer Wärme-Kraftanlage vor der Inbetriebnahme über den hochaktiven Adsorptionsfilter zirkulieren lässt. Der möglicherweise erschöpfte Adsorber kann zwischenzeitlich durch einen frischen Adsorber ausgetauscht werden. Eine analoge Arbeitsweise ist auch bei einem evtl. Nachfüllen der Anlage mit frischen FKW vorstellbar. Auf diese Art und Weise wird sichergestellt, dass alle mit dem Arbeitsmedium in Berührung kommenden Teile wasserfrei sind. Die Anwendung dieser Methode schliesst aber nicht aus, hochtrockene FKW mit Restwassergehalten kleiner 1 ppm einzusetzen, nachdem die Anlage mit Fluorchlorkohlenwasserstoffen zuvor gespült worden ist, wodurch ebenfalls eine bedeutende Herabsetzung des $H_2O$-Gehaltes im Arbeitsmedium erzielt wird.

Ein weiterer, zusätzlicher Effekt des Adsorptionsfilters liegt in der Tatsache begründet, dass feine Rostpartikel und Schmutzteilchen durch die Adsorberschicht ausgefiltert werden. Bevorzugt Eisenoxide wandeln sich durch HCl und HF schnell in die katalytisch wirkenden Eisenhalogenide um, die ihrerseits, wenn sie im Arbeitsmittel gelöst sind, durch den Adsorber fixiert werden.

2. Entfernung der aciden Bestandteile Chlorid und Fluorid. In einer kontinuierlich betriebenen Wärmekraftmaschine oder Wärmepumpe ist durch eine kurzfristige Übertemperatur, z.B. im Rauchgas, nicht auszuschliessen, dass in dieser Zeit eine geringfügige Thermolyse des Arbeitsmediums Fluorchlorkohlenwasserstoff eintritt. Um Korrosionserscheinungen im System zu vermeiden, ist es notwendig, die dafür verantwortlichen aciden Bestandteile Fluorid und Chlorid aus dem Arbeitsmedium zu entfernen. Es wurde nun gefunden, dass diese wichtige Funktion von einem basischen Adsorptionsmittel vorteilhaft bewirkt wird. Als basische Adsorptionsmittel kommen basische Oxide, insbesondere Oxide der Erdalkalimetalle oder des Zinks in Frage. In einer anderen Variante kann aber auch basisches Aluminiumoxid, insbesondere basisches $Al_2O_3$ für Säulenchromatographie verwendet werden. Es ist zwar bereits aus der DE-A 23 10 749 bekannt, zur Entfernung von Fluorwasserstoff organische Fluorverbindungen über aktives Aluminiumoxid zu leiten. Das dort verwendete Aluminiumoxid ist aber neutral. Es war zu befürchten, bei Verwendung von basischem Aluminiumoxid dem Kreislaufmittel zusätzlich durch Neutralisation entstehendes Wasser zuzuführen und somit die gestellte Aufgabe «Entfernung von Wasser» zu erschweren. Ferner kann dem Stand der Technik keine Aussage über die Entfernung anderer acider oder katalytisch wirkender Komponenten entnommen werden und es war zudem zu befürchten, dass das Arbeiten in kondensierter Phase zu einer verminderten Entfernung von Fluorwasserstoff führt.

Durch die erfindungsgemässe Adsorptions-Anordnung wird in Wärmekraftanlagen über lange Zeiträume hinweg ein pH-neutrales Kreislaufmedium aufrechterhalten.

Eine erhebliche Steigerung dieses Neutralisationseffektes wird erzielt, wenn man in der Form eines Mischbettes zu dem hochaktiven Adsorptionsmittel das basische Adsorptionsmittel zusetzt. Es ist aber dafür Sorge zu tragen, dass das bei der Reaktion z.B. von Calcium-Oxid mit z.B. HF und HCl gebildete Wasser von einer nachfolgenden Schicht hochaktivem Aluminiumoxid sicher adsorbiert wird.

In dieser Anordnung kann die Entwässerung auch durch hochaktive Adsorptionsmittel auf Basis $SiO_2$ erreicht werden, ohne dass dabei Si-Halogenverbindungen entstehen.

Jeder erfindungsgemässe Teilaspekt bewirkt für sich allein bereits eine wesentliche Verbesserung der thermischen Stabilität von Fluor-Chlor-Kohlenwasserstoffen insbesondere der Verbindungen R 11, 12, 22, 113, 114, 115.

Eine zusätzliche Sicherheit lässt sich durch Passivierung aller thermisch hochbelasteten eisenhaltigen Apparateteile, die in Kontakt auf dem Fluor-Chlor-Kohlenwasserstoff stehen, durch Heissnitridierung erreichen. Diese Massnahme ist als solche z.B. aus DD-A-93 685 bekannt.

Die Nitridierung von Stahl oder Stahllegierungen erfolgt dabei mit Ammoniak (Gas) bei Temperaturen oberhalb 500 °C, vorzugsweise bei 500 bis 800 °C. Hier wird durch die Bildung von Eisennitrid auf der Metalloberfläche ein Passivierungseffekt gegenüber den Fluorchlorkohlenwasserstoffen erreicht. Von entscheidender Bedeutung ist, dass die Oberflächenpassivierung durch ein thermisches Nitridierungsverfahren erfolgt. Damit wird gewährleistet, dass die Oberfläche des nitridierten Stahls bei den herrschenden Arbeitstemperaturen nicht durch die thermisch bedingte Materialausdehnung partiell zerstört wird und dadurch Ansatzpunkte für eine Korrosion bietet.

So konnte die Zersetzungstemperatur im Falle von R 11 als Arbeitsmedium in Kohlenstoffstahl (St 39) durch eine Nitridierung der heissgasführenden Apparateteile und Leitungen von 170 °C auf 210 °C angehoben werden. Durch die Nitridierung von Edelstahl (St 4571) von 210 auf 220 °C. Damit ist festgestellt, dass auch Stahlsorten mit geringerem Eisengehalt durch eine Nitridierung hinsichtlich des thermischen Stabilitätsverhaltens von Fluor-Chlor-Kohlenwasserstoffen eine Verbesserung bewirken. Preisgünstige Kohlenstoff-Stähle, wie sie in diesem Gebiet des Apparatebaues eingesetzt werden, werden auf das Niveau von Edelstählen angehoben. Diese Massnahme reicht aber allein noch nicht aus, um im Dauerbetrieb bei Temperaturen über 200 °C zu arbeiten.

Durch die völlige Entfernung von Wasser aus den Fluorkohlenwasserstoffen mit hochaktivem $Al_2O_3$ (basisch) für Säulenchromatographie kann die Zersetzungstemperatur bei Verwendung von R 11 in unbehandeltem C-Stahl von 170 °C auf 250 °C heraufgesetzt werden, ohne dass über längere Zeiträume hinweg eine Veränderung am Arbeitsmedium festgestellt werden konnte.

Die Entfernung der aciden Verbindung HF und HCl aus dem Kreislaufmedium R 11, das kurzfristig einer Verdampfertemperatur von 290 °C ausgesetzt war, erfolgte durch den erfindungsgemässen Einsatz von hochaktivem $Al_2O_3$-basisch für Säulenchromatographie vollständig. Das R 11 reagierte neutral. Dadurch konnte ein Austausch des Arbeitsmediums vermieden werden, was in der technischen Anwendung eine erhebliche Kostenersparnis bedeutet.

Durch eine Kombination der einzelnen erfindungsgemässen Teilaspekte konnte die Zersetzungstemperatur für R 11 in Kohlenstoffstahl (St 39) von 170 °C auf 270 °C in einer Versuchsanlage, in der die echten Betriebsbedingungen, bezogen auf Druck- und Temperaturhöhe, Druck- und Temperaturwechsel, simuliert wurden, gesteigert werden.

Durch das erfindungsgemässe Verfahren zur Verbesserung des thermischen Stabilitätsverhaltens von FKW's ist man nunmehr in der Lage, die vielerorts angebotene Abwärme von 80 bis 300 °C, in der Wasser nicht mehr wirtschaftlich als Arbeitsmedium im Rankine-Prozess eingesetzt werden kann, zur Gewinnung von elektrischer Energie oder mechanischer Antriebskraft zu nutzen. Die Anwendungszwecke beschränken sich nicht nur auf die Gewinnung von elektrischem Strom und mechanischer Antriebsenergie. Es ist nunmehr auch möglich, Wärmepumpen zu bauen, die über den heutigen Stand der Technik hinaus z.B. Wasser oder Wasserdampf von mehr als 150 °C produzieren.

An Hand der nachfolgenden Beispiele soll eine Erläuterung des erfindungsgemässen Verfahrens gegeben werden.

a. Versuchsapparatur

Die Untersuchungen über das erfindungsgemässe Verfahren über die Verbesserung des thermischen Stabilitätsverhaltens von Fluor-Chlor-Kohlenwasserstoffen, insbesondere der Typen R 11, 12, 22, 113, 114, 115 wurden in einer dynamischen Versuchsanlage durchgeführt, in der die echten Betriebsbedingungen, bezogen auf Temperatur und Druck, Temperatur- und Druckwechsel, sowie den Einsatz verschiedener Konstruktionsmaterialien, Ölen, Dichtungen simuliert wurden.

Die Versuchsapparatur ist in der Art einer Kompaktanlage aufgebaut, in der der Kreisprozess wie folgt abläuft.

Das Kreislaufmedium wird dem Kondensatsammler 1 entnommen und über ein mechanisches Filter 2 der Speisepumpe 3 zugeführt. Parallel zu der Speiseleitung 4 befindet sich in einer By-pass-Leitung 5 der Adsorptionsfilter 6 mit der hochaktiven Aluminiumoxidfüllung – basisch – für Säulenchromatographie (Produkt der Fa. Woelm). Durch diese Anordnung ist es möglich, das Kreislaufmittel direkt oder über den Adsorptionsfilter zur Speisepumpe 3 zu führen. Die Speisepumpe fördert das Wärmeträgermedium – entsprechend dem vorgegebenen Druck und der Durchflussmenge – in den Verdampfer 7. Diese Verdampferrohrschlange befindet sich in einem elektrisch beheizten Ölbad. Das verdampfte Arbeitsmedium wird dann einem pneumatischen Regelventil 8 zugeführt, das die Funktion einer Turbine oder eines Gasmotors simuliert. Nach der Entspannung gelangt das Arbeitsmedium in den Kondensator 9, eine wassergekühlte Rohrschlange, wird dort kondensiert und in dem Kondensatsammelbehälter 1 aufgefangen.

Die Umlaufmenge des Arbeitsmediums betrug 36 kg/h. Vor einer jeden Versuchsreihe wurde die gesamte Anlage einer sorgfältigen Reinigunsoperation unterzogen.

b. Nitridierung

Die Nitridierung des Rohrverdampfers sowie aller Heissdampf führenden Leitungen und Ventile wurde nach folgendem Schema durchgeführt.

Die zum Einsatz kommenden Teile aus Kohlenstoffstahl oder aus Stahl 4571 wurden zunächst gründlich gesäubert und entfettet. Nach dem Trocknen wurde durch die Teile in einem Ofen bei einer Temperatur von 600 bis auf 750°C ansteigend Ammoniak und Stickstoff (letzteres als Inertgas) im Volumenverhältnis 2:1 und einem Durchsatz von 15 l/h durchgeleitet. Zur Kontrolle des Nitridierungsgrades waren Proberohre eingefügt worden.

Nach 8 Stunden war das Eisen an der Metalloberfläche in $\gamma$-Eisennitrid überführt worden (Röntgenfeinstrukturanalyse). Anschliessend wurden die Teile in die Testapparaturen eingefügt.

Die Kontrolle des Arbeitsmediums erfolgte
1 durch die analytische Bestimmung des Fluorid- und Chlorid-Gehaltes und
2 durch Gaschromatographie.

Die Analysenproben wurden nach dem Verdampfer, vor der Speisepumpe und nach dem $Al_2O_3$-Adsorber gezogen.

c. Beispiele
Beispiel 1

In der beschriebenen Versuchsapparatur (nicht nitridiert) wurde zunächst der Einfluss verschiedener Konstruktionsmaterialien auf das thermische Zersetzungsverhalten von R 11 und R 113 in Abhängigkeit von der Temperatur untersucht. Der Arbeitsmittelkreislauf betrug 36 kg/h. Der Druck wurde entsprechend der Dampfdruckkurve minus 10 % – um Kondensation auszuschliessen – der jeweiligen Verdampfertemperatur angepasst. In Tabelle 1 wird der Gehalt an Fluorid und Chlorid im Heissgasstrom in Abhängigkeit von der Temperatur sowie die gaschromatogrphischen Analysen angegeben. Die Versuchszeit pro Temperatureinstellung betrug 180 Betriebsstunden.

| Temperatur des Heissgases nach Verdampfer °C | C-Stahl St 39 | | | | Edelstahl St 4571 | | | | Edelstahl St 4309 | | | |
|---|---|---|---|---|---|---|---|---|---|---|---|---|
| | Gehalt ppm | | GC F-% | | Gehalt ppm | | GC F-% | | Gehalt ppm | | GC F-% | |
| R 11 | F′ | Cl′ | R 11 | Rest | F′ | Cl′ | R 11 | Rest | F′ | Cl′ | R 11 | Rest |
| 20 | 0,1 | 0,1 | 99,99 | 0,01 | 0,1 | 4,0 | 99,98 | 0,02 | 0,1 | 3,1 | 99,98 | 0,02 |
| 150 | 0,1 | 0,5 | 99,99 | 0,01 | | | | | 0,7 | 2,1 | 99,98 | 0,02 |
| 170 | 0,1 | 0,5 | 99,99 | 0,01 | 0,1 | 2,9 | 09,98 | 0,02 | 0,1 | 0,2 | 99,98 | 0,02 |
| 180 | 0,1 | 0,5 | 99,99 | 0,01 | 0,1 | 0,8 | 99,98 | 0,02 | 0,1 | 0,1 | 99,97 | 0,03 |
| 190 | 1,0 | 2,8 | 99,98 | 0,02 | 0,1 | 0,7 | 99,98 | 0,02 | 0,1 | 0,2 | 99,96 | 0,04 |
| 200 | 1,4 | 12,9 | 99,97 | 0,02 | 0,1 | 0,7 | 99,98 | 0,03 | 0,2 | 0,6 | 99,96 | 0,04 |
| 210 | 1,7 | 4,5 | 99,97 | 0,03 | 0,1 | 0,5 | 99,97 | 0,03 | 0,2 | 0,6 | 99,96 | 0,04 |
| 220 | | | | | 0,3 | 1,8 | 99,98 | 0,02 | 0,1 | 0,5 | 99,96 | 0,04 |
| 230 | | | | | 3,0 | 17,7 | 99,97 | 0,02 | 0,9 | 4,5 | 99,96 | 0,04 |
| 240 | | | | | 16,0 | 207,0 | 99,95 | 0,05 | 1,4 | 11,2 | 99,70 | 0,30 |
| 250 | | | | | | | | | | | | |
| R 113 | F′ | Cl′ | R 113 | Rest | F′ | Cl′ | R 113 | Rest | F′ | Cl′ | R 113 | Rest |
| 20 | 0,1 | 0,2 | 99,98 | 0,02 | 0,1 | 0,3 | 99,98 | 0,02 | 0,1 | 0,3 | 99,98 | 0,02 |
| 120 | 0,1 | 0,2 | 99,98 | 0,02 | 0,1 | 0,7 | 99,98 | 0,02 | | | | |
| 140 | | | | | | | | | | | | |
| 150 | 0,0 | 0,2 | 99,97 | 0,03 | 0,4 | 0,9 | 99,98 | 0,02 | | | | |
| 160 | 0,5 | 1,6 | 99,97 | 0,03 | 0,6 | 1,2 | 99,96 | 0,04 | 0,6 | 0,3 | 99,97 | 0,03 |

| R 113 | F′ | Cl′ | R 113 | Rest | F′ | Cl′ | R 113 | Rest | F′ | Cl′ | R 113 | Rest |
|---|---|---|---|---|---|---|---|---|---|---|---|---|
| 170 | 0,4 | 1,7 | 99,97 | 0,03 | 0,6 | 1,2 | 99,97 | 0,03 | 0,6 | 0,3 | 99,97 | 0,03 |
| 180 | 1,0 | 10,7 | 99,92 | 0,08 | 0,7 | 1,5 | 99,97 | 0,03 | 0,1 | 0,9 | 99,97 | 0,03 |
| 190 | 3,0 | 17,0 | 99,84 | 0,16 | 0,9 | 1,8 | 99,96 | 0,04 | 0,9 | 2,4 | 99,97 | 0,03 |
| 200 | | | | | 4,0 | 15,1 | 99,98 | 0,11 | 2,0 | 7,0 | 99,93 | 0,07 |
| 210 | | | | | | | | | | | | |
| 220 | | | | | | | | | | | | |
| 230 | | | | | | | | | | | | |

## Beispiel 2

In der beschriebenen Versuchsapparatur wurde der Einfluss von nitridiertem C-Stahl sowie Stahl 4571 (wie in b hergestellt) auf das thermische Zersetzungsverhalten von R 11 und R 113 untersucht. Die Versuchsbedingungen entsprachen denen im Beispiel 1.

Tabelle 2

| Temperatur des Heissgases nach Verdampfer °C | C-Stahl nitridiert ST 39 | | | | Edelstahl nitridiert St 4571 | | | |
|---|---|---|---|---|---|---|---|---|
| | Gehalt ppm | | GC F-% | | Gehalt ppm | | GC F-% | |
| | F′ | Cl′ | R 11 | Rest | F′ | Cl′ | R 11 | Rest |
| 20 | 0,1 | 2,6 | 99,97 | 0,03 | 0,1 | 2 | 99,98 | 0,02 |
| 170 | 0,1 | 0,7 | 99,96 | 0,06 | | | | |
| 180 | 0,1 | 0,3 | 99,96 | 0,06 | 0,1 | 0,1 | 99,98 | 0,02 |
| 190 | 0,1 | 0,7 | 99,96 | 0,06 | 0,7 | 2,8 | 99,98 | 0,02 |
| 200 | 0,1 | 0,5 | 99,96 | 0,06 | 0,1 | 1,4 | 99,96 | 0,04 |
| 210 | 0,1 | 0,6 | 99,96 | 0,06 | 0,2 | 2,1 | 99,96 | 0,04 |
| 220 | 0,5 | 1,5 | 99,96 | 0,06 | 0,1 | 0,8 | 99,96 | 0,04 |
| 230 | 2,6 | 11,6 | 99,95 | 0,05 | 0,5 | 2,3 | 99,96 | 0,04 |
| 240 | | | | | 5,8 | 14,7 | 99,91 | 0,01 |

| R 113 | F′ | Cl′ | R 133 | Rest | F′ | Cl′ | R 113 | Rest |
|---|---|---|---|---|---|---|---|---|
| 20 | 0,1 | 0,4 | 99,98 | 0,02 | | | | |
| 170 | 0,4 | 0,2 | 99,98 | 0,02 | | | | |
| 190 | 0,2 | 3,1 | 99,97 | 0,03 | | | | |
| 200 | 0,5 | 1,7 | 99,98 | 0,02 | | | | |
| 210 | 0,8 | 2,7 | 99,96 | 0,04 | | | | |
| 220 | 1,1 | 4,8 | 99,96 | 0,04 | | | | |
| 230 | 4,3 | 16,1 | 99,92 | 0,08 | | | | |

## Beispiel 3

In der beschriebenen Versuchsapparatur (nicht nitridiert) wurden als Verdampferschlage und als Heissgas führendes Leitungsrohr Normalstahl (St 39) eingesetzt. Das Arbeitsmedium R 11 konnte wahlweise aus dem Kondensatsammler direkt zur Speisepumpe fliessen oder im By-pass über einen Aluminiumoxid-Adsorber geführt werden. Als Adsorber diente ein Glasbehälter mit einem Volumen von 300 ml-Inhalt. In diesem befanden sich 200 g Aluminiumoxid-basisch für Säulenchromatographie der Fa. Woelm. Das Arbeitsmittel wurde oben auf die $Al_2O_3$-Schüttung aufgegeben und am Adsorberboden abgezogen. Es passierte ein Feinfilter und einen Abscheider und gelangte von dort zur Speisepumpe. Nach dem Befüllen der Anlage wurde zunächst das Arbeitsmedium 75 Stunden bei 20 °C über den Adsorber gepumpt, um eine vollständige Trocknung des Arbeitsmediums und der mit dem Arbeitsmedium in Berührung stehenden Apparateteile zu erreichen. Anschliessend wurde die Adsorberkartusche gegen eine frische Kartusche ausgetauscht und das System auf Arbeitstemperatur gebracht. Die Anlage wurde so betrieben, dass vor Beginn einer jeden Temperatureinstellung das Arbeitsmedium 2 Stunden lang über den Adsorber geleitet wurde. In der Tabelle 3 werden der Gehalt an aciden Bestandteilen F′ und Cl′ und die Veränderung in der Gaszusammensetzung in Abhängigkeit von der Temperatur angegeben.

Tabelle 3

| Temperatur des Heissgases nach Verdampfer °C | R 11 | | | | R 113 | | | |
|---|---|---|---|---|---|---|---|---|
| | Gehalt ppm F′ | Cl′ | GC F-% R 11 | Rest | Gehalt ppm F′ | Cl′ | GC F-% R 113 | Rest |
| 20 | 0,7 | 1,0 | 99,98 | 0,02 | 0,3 | 0,6 | 99,98 | 0,02 |
| 170 | 0,9 | 9,4 | 99,98 | 0,02 | 0,1 | 1,2 | 99,98 | 0,02 |
| 180 | 0,1 | 1,3 | 99,98 | 0,02 | 0,1 | 0,9 | 99,98 | 0,02 |
| 190 | 0,1 | 1,5 | 99,97 | 0,03 | 0,1 | 0,3 | 99,98 | 0,02 |
| 200 | 0,1 | 0,3 | 99,98 | 0,02 | 0,1 | 0,3 | 99,98 | 0,02 |
| 210 | 0,1 | 0,1 | 99,98 | 0,02 | 0,1 | 0,1 | 99,96 | 0,04 |
| 220 | 0,1 | 0,1 | 99,98 | 0,02 | 0,1 | 0,2 | 99,97 | 0,03 |
| 230 | 0,1 | 0,1 | 99,97 | 0,03 | 0,2 | 1,0 | 99,95 | 0,05 |
| 240 | 0,2 | 0,1 | 99,96 | 0,04 | 1,1 | 3,4 | 99,78 | 0,22 |
| 250 | 0,5 | 0,6 | 99,96 | 0,04 | | | | |
| 260 | 1,9 | 17,5 | 99,82 | 0,08 | | | | |
| 270 | 3,1 | 61,6 | 99,88 | 0,12 | | | | |
| 280 | | | | | | | | |
| 290 | | | | | | | | |

Beispiel 4

In der beschriebenen Versuchsapparatur wurden als Verdampferschlange und als Heissgas führendes Leitungsrohr Normalstahl (St 39) nitridiert eingesetzt. Die Versuchsdurchführung erfolgte mit R 11 als Arbeitsmedium nach Beispiel 3 mit eingebautem Adsorber. Die R 11-Zirkulation vor dem Temperaturprogramm wurde von 75 h auf 16 h reduziert.

In der Tabelle 4 wird der Zersetzungsbeginn des Kreislaufmediums tabellarisch aufgezeigt.

Tabelle 4

| Temperatur des Heissgases nach Verdampfer °C | Arbeitsmittel R 11 | | | |
|---|---|---|---|---|
| | Gehalt ppm F′ | Cl′ | GC F-% R 11 | Rest |
| 20 | 0,1 | 0,2 | 99,98 | 0,02 |
| 180 | 0,3 | 0,7 | 99,98 | 0,02 |
| 200 | 0,1 | 0,6 | 99,98 | 0,02 |
| 210 | 0,1 | 0,1 | 99,96 | 0,04 |
| 220 | 0,1 | 0,2 | 99,97 | 0,03 |
| 230 | 0,1 | 0,1 | 99,97 | 0,03 |
| 240 | 0,1 | 0,1 | 99,97 | 0,03 |
| 250 | 0,1 | 0,6 | 99,96 | 0,03 |
| 260 | 0,2 | 0,6 | 99,96 | 0,04 |
| 270 | 1,2 | 4,8 | 99,93 | 0,07 |

Beispiel 5

In der beschriebenen Versuchsapparatur wurde während eines gemäss Beispiel 3 dargestellten Versuches das Adsorptionsverhalten des basischen hochaktiven $Al_2O_3$ für Säulenchromatographie gegenüber den aciden Bestandteilen HF und HCl im Kreislaufmedium untersucht. Während einer Zirkulationsphase, bei der das Kreislaufmedium den Adsorptionsfilter durchlief, wurden vor und hinter dem Adsorber Proben gezogen und darin die Acidität bestimmt. In der Tabelle 5 werden die Gehalte an Fluorid und Chlorid vor und nach der Adsorption angegeben:

Tabelle 5

| Adsorptions- dauer (h) | Gehalt an Fluorid und Chlorid | | | |
|---|---|---|---|---|
| | vor Adsorption (ppm) F′ | Cl′ | nach Adsorption (ppm) F′ | Cl′ |
| 0 | 6,8 | 26,4 | <0,1 | <0,3 |
| 1 | 0,4 | 2,1 | <0,1 | <0,3 |
| 2 | 0,6 | 0,5 | <0,1 | <0,3 |
| 3 | 0,2 | <0,3 | <0,1 | <0,3 |
| 4 | 0,1 | <0,3 | <0,1 | <0,3 |

Beispiel 6

In der beschriebenen Versuchsapparatur (nicht nitridiert) wurde während eines gemäss Beispiel 3 dargestellten Versuches das Adsorptionsverhalten eines Adsorbers, bestehend aus hochaktivem $Al_2O_3$ für Säulenchromatographie und verschiedenen Erdalkalioxiden gegenüber den aciden Bestandteilen HF und HCl im Kreislaufmedium untersucht. Das Adsorptionsfilter bestand aus 190 g $Al_2O_3$ und 10 g Magnesiumoxid, wobei letzteres sich im Eingangsteil des Adsorbers befand.

Tabelle 6

| Adsorptions- dauer (h) | Gehalt an Fluorid und Chlorid | | | |
|---|---|---|---|---|
| | vor Adsorption (ppm) F′ | Cl′ | nach Adsorption (ppm) F′ | Cl′ |
| 0 | 6,8 | 26,4 | <0,1 | <0,3 |
| 1 | 0,2 | 1,2 | <0,1 | <0,3 |
| 2 | <0,1 | <0,3 | <0,1 | <0,3 |
| 3 | <0,1 | <0,3 | <0,1 | <0,3 |

Die Adsorptionskapazität erhöhte sich bei einer Adsorber-Zusammensetzung von jeweils 190 g

| | | | |
|---|---|---|---|
| $Al_2O_3$ | + | – | 100,0% |
| $Al_2O_3$ | + | MgO | 100,0% |
| $Al_2O_3$ | + | BaO | 118 % |
| $Al_2O_3$ | + | CaO | 102 % |
| $Al_2O_3$ | + | ZNO | 108 % |

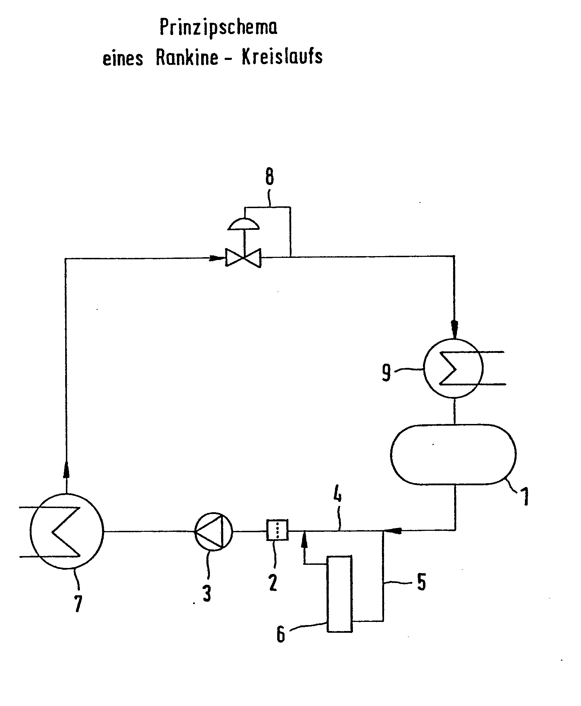

## Patentansprüche

1. Verfahren zur Vermeidung von Korrosionsschäden in thermisch hochbelasteten Rankine-Prozess-Anlagen mit aliphatischen Fluor-Chlor-Kohlenwasserstoffen als Kreislaufmittel, dadurch gekennzeichnet, dass man das Kreislaufmittel in kondensierter Form über ein hochaktives Adsorptionsmittel für Wasser auf Basis von $Al_2O_3$ und/oder $SiO_2$ und ein basisches Adsorptionsmittel für acide Verbindungen leitet.

2. Verfahren nach Anspruch 1, dadurch gekennzeichnet, dass das basische Adsorptionsmittel Oxide der Erdalkalimetalle oder des Zinks enthält.

3. Verfahren nach Anspruch 1, dadurch gekennzeichnet, dass das basische Adsorptionsmittel basisches $Al_2O_3$ ist.

4. Verfahren nach einem der Ansprüche 1 bis 3, dadurch gekennzeichnet, dass man das Kreislaufmittel vollständig oder in einem Teilstrom über die Adsorptionsmittel leitet.

5. Verfahren nach einem der Ansprüche 1–4, dadurch gekennzeichnet, dass man das Kreislaufmittel kontinuierlich oder diskontinuierlich über die Adsorptionsmittel leitet.

6. Verfahren nach einem der vorhergehenden Ansprüche, dadurch gekennzeichnet, dass man zusätzlich alle thermisch hochbelasteten, eisenhaltigen Apparateteile, die in Kontakt mit dem Kreislaufmittel stehen, durch Heissnitridierung passiviert.

7. Verfahren nach Anspruch 6, dadurch gekennzeichnet, dass man die Heissnitridierung mit einem Gemisch aus Ammoniak und Stickstoff durchführt.

8. Verfahren nach Anspruch 6 oder 7, dadurch gekennzeichnet, dass man die Heissnitridierung bei Temperaturen oberhalb von 500 °C, vorzugsweise bei 500 bis 800 °C durchführt.

9. Anlage zur Ausführung eines Rankine-Prozesses mit einem Kondensator, Fördermittel und/oder Arbeitsglied und Verdampfer, mit Fluor-Chlor-Kohlenwasserstoffen als Kreislaufmittel, zur Durchführung eines Verfahrens gemäss einem der vorstehenden Ansprüche, dadurch gekennzeichnet, dass zwischen Kondensator (9) und Verdampfer (7) ein Adsorptionsfilter (6) vorgesehen ist, und gegebenenfalls alle thermisch hochbelasteten, eisenhaltigen Apparateteile, die in Kontakt mit dem Kreislaufmittel stehen, durch Heissnitridierung passiviert sind.

## Claims

1. Method for avoiding corrosion damage in thermally highly loaded Rankine process installations with aliphatic fluoro-chloro-hydrocarbons as circulating medium, characterized in that the circulating medium in condensed form is conducted over a highly active adsorption medium for water which is based on $Al_2O_3$ and/or $SiO_2$ and a basic adsorption medium for acidic compounds.

2. Method according to Claim 1, characterized in that the basic adsorption medium contains oxides of alkaline earth metals or of zinc.

3. Method according to Claim 1, characterized in that the basic adsorption medium is basic $Al_2O_3$.

4. Method according to one of Claims 1 to 3, characterized in that all or a partial stream of circulating medium is conducted over the adsorption medium.

5. Method according to one of Claims 1–4, characterized in that the circulating medium is conducted continuously or discontinuously over the adsorption medium.

6. Method according to one of the preceding Claims, characterized in that in addition all thermally highly loaded, ferrous parts of the apparatus, which are in contact with the circulating medium, are rendered passive by hot nitriding.

7. Method according to Claim 6, characterized in that the hot nitriding is carried out with a mixture of ammonia and nitrogen.

8. Method according to Claim 6 or 7, characterized in that the hot nitriding is carried out at temperatures above 500 °C, preferably at 500 to 800 °C.

9. Installation for carrying out a Rankine process with a condenser, transport means and/or working member and evaporator, with fluoro-chloro-hydrocarbons as circulating medium, for carrying out a method according to one of the above claims, characterized in that between the condenser (9) and evaporator (7) an adsorption filter (6) is provided, and if required all thermally highly loaded, ferrous parts of the apparatus, which are in contact with the circulating medium are rendered passive through hot nitriding.

## Revendications

1. Procédé pour la prévention des dommages causés par la corrosion dans des appareillages pour procédé Rankine soumis à une forte contrainte thermique contenant des hydrocarbures chloro-fluorés comme agent de circulation, caractérisé en ce que l'on fait passer l'agent de circulation à l'état condensé sur un adsorbant hautement actif à base d'$Al_2O_3$ et/ou de $SiO_2$ pour l'eau et sur un adsorbant basique pour les composés acides.

2. Procédé selon la revendication 1, caractérisé en ce que l'adsorbant basique renferme des oxydes de métaux alcalino-terreux ou de zinc.

3. Procédé selon la revendication 1, caractérisé en ce que l'adsorbant basique est de l'$Al_2O_3$ basique.

4. Procédé selon l'une des revendications 1 à 3, caractérisé en ce qu'on fait passer l'agent de circulation entièrement ou en un courant partiel sur l'adsorbant.

5. Procédé selon l'une des revendications 1 à 4, caractérisé en ce qu'on fait passer l'agent de circulation en continu ou en discontinu sur l'adsorbant.

6. Procédé selon l'une des revendications précédentes, caractérisé en ce qu'on passive en outre par nitruration à chaud tous les éléments de l'appareil contenant du fer soumis à une forte contrainte thermique qui se trouvent en contact avec l'agent de circulation.

7. Procédé selon la revendication 8, caractérisé en ce qu'on effectue la nitruration à chaud avec un mélange d'ammoniac et d'azote.

8. Procédé selon la revendication 6 ou 7, caractérisé en ce qu'on effectue la nitruration à chaud à des températures au-dessus de 500 °C, de préférence entre 500 et 800 °C.

9. Appareillage pour la mise en œuvre d'un procédé Rankine avec un condenseur, un véhicule et/ou un maillon opératoire et un vaporiseur, des hydrocarbures chloro-fluorés comme agent de circulation, pour la mise en œuvre d'un procédé selon l'une des revendications précédentes, caractérisé en ce qu'un filtre d'adsorption (6) est prévu entre le condenseur (9) et le vaporiseur (7) et qu'éventuellement tous les éléments de l'appareil contenant du fer, soumis à une forte contrainte thermique, qui se trouvent en contact avec l'agent de circulation, sont passivés par nitruration à chaud.

# Prinzipschema
## eines Rankine - Kreislaufs